(19) Europäisches Patentamt
European Patent Office
Office européen des brevets

(11) **EP 2 528 457 B1**

## (12) EUROPEAN PATENT SPECIFICATION

(45) Date of publication and mention
of the grant of the patent:
**14.01.2015 Bulletin 2015/03**

(21) Application number: **11705725.7**

(22) Date of filing: **28.01.2011**

(51) Int Cl.:
*A61L 2/07* (2006.01)　　*A61L 2/18* (2006.01)
*A61L 2/22* (2006.01)　　*B65B 55/10* (2006.01)
*B65B 55/18* (2006.01)　　*A23L 1/30* (2006.01)
*A23L 1/29* (2006.01)

(86) International application number:
**PCT/US2011/022938**

(87) International publication number:
**WO 2011/094551 (04.08.2011 Gazette 2011/31)**

(54) **PLASTIC PACKAGED NUTRITONAL LIQUIDS COMPRISING HMB**

IN KUNSTSTOFFPACKUNGEN VERPACKTE NAHRUNGSFLÜSSIGKEITEN MIT HMB

LIQUIDES NUTRITIONNELS COMPRENANT DU MHB EMBALLÉS DANS UN EMBALLAGE EN PLASTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.01.2010 US 299718 P**

(43) Date of publication of application:
**05.12.2012 Bulletin 2012/49**

(73) Proprietor: **ABBOTT LABORATORIES**
**Abbott Park, IL 60064-3500 (US)**

(72) Inventors:
• **JOHNS, Paul, W**
  **Columbus, Ohio 43221 (US)**
• **KENSLER, Ann**
  **Sugar Grove, Ohio 43155 (US)**

(74) Representative: **Modiano, Micaela Nadia et al**
**Modiano & Partners (DE)**
**Thierschstrasse 11**
**80538 München (DE)**

(56) References cited:
**US-A1- 2005 215 640**

• **KREIDER R B ET AL: "EFFECT OF CALCIUM BETA-HYDROXY-BETA-METHYLBUTYRATE (HMB) SUPPLEMENTATION DURING RESISTANCE-TRAINING ON MARKERS OF CATABOLISM, BODY COMPOSITION AND STRENGTH", INTERNATIONAL JOURNAL OF SPORTS MEDICINE, THIEME, STUTTGART, DE, vol. 20, no. 8, 1 November 1999 (1999-11-01), pages 503-509, XP008037671, ISSN: 0172-4622, DOI: DOI:10.1055/S-1999-8835**

EP 2 528 457 B1

**Description**

**FIELD OF THE DISCLOSURE**

The present disclosure relates to plastic packaged nutritional liquids comprising beta-hydroxy-beta-methylbutyrate (HMB).

**BACKGROUND OF THE DISCLOSURE**

There are many different types of packaged nutritional liquids suitable for oral administration to humans, which compositions typically comprise various combinations of macro and micro nutrients. Many of these packaged liquids are formulated as milk or protein-based emulsions for use as sole or supplemental sources of nutrition. These packaged emulsions are often manufactured as oil-in-water emulsions comprising fat, protein, carbohydrate, vitamins, and minerals, some examples of which include ENSURE® Nutritional Liquid and GLUCERNA® Shakes available from Abbott Laboratories, Columbus, Ohio USA.

US 2005/215640 A1 discloses beta-hydroxy-beta-methylbutyrate (HMB), either alone or in combination with other compounds, for use in preventing and treating some conditionsand diseases such as, among others, chronic inflammatory diseases, cancer, cachexia. US 2005/215640 A1 also discloses addition of HMB to food products: in particular, a liquid composition comprising HMB and carbohydrates, filled into plastic bottles and retorted to food grade standards is described in this document.

During the manufacturing process, these packaged nutritional compositions are sterilized to reduce microbial contaminants to the extent necessary to render the compositions suitable for oral administration to humans. These processes often include thermal processes such as retort sterilization and aseptic process sterilization. A typical retort process involves introducing the nutritional composition into a suitable container, sealing the container, and then heating the sealed container and its contents for a time period and at temperature sufficient for sterilization. An aseptic sterilization process on the other hand typically involves separately sterilizing the interior of a food grade container and a nutritional composition and then combining the sterilized container and the sterilized nutritional composition in a clean room environment and sealing the container.

There are many different types of containers and container materials that are suitable for processing and packaging nutritional liquids such as shelf-stable nutritional emulsions and other liquids. Such containers are designed to accommodate elevated processing temperatures associated with many different types of sterilization processes. These containers include glass, aluminum or other metals, paper, plastic or other polymer materials, various laminates, and combinations thereof, many of which further comprise other container materials to provide for the safe and effective packaging of the nutritional liquid over the desired shelf life. Among these many choices, plastic containers have become increasingly popular among consumers and manufacturers as a convenient, cost effective, and light weight container for many different types of nutritional liquids.

Although plastic containers are commercially advantageous and have been used in the past for the packaging of nutritional liquids, including nutritional emulsions prepared by either retort or aseptic processing methods, plastic packaging has traditionally suffered from a number of shortcomings such that its use has been generally limited. For example, nutritional liquids in plastic packages are subject to a more pronounced drop in pH over time as compared to nutritional liquids packaged in metal, glass or similar other materials. This reduction in pH of the product over time in plastic containers may be attributed to at least three oxidation-enhancing factors, all of which can increase the oxidation of the nutritional liquids contained in the plastic container and result in a reduction in pH over time.

First, plastic containers are often subjected to more severe heat treatment during retort sterilization as compared to metal containers since plastic is less conductive of heat than metal and so increased temperatures are required with plastic to achieve the intended sterilization result. This more severe heat treatment can result in increased oxidation of the nutritional liquid contained inside the container, especially when the nutritional product contains fat or other easily oxidized ingredients.

Second, plastic containers often contain a larger headspace volume as compared to many metal containers, which means there is a larger volume of air or gas present in plastic containers as compared to metal containers. Generally, a plastic container will often have about two to three times the headspace volume as compared to an equally-sized metal container. This increased volume of headspace air or gas can lead to increased oxidation of the nutritional liquid within the container.

Third, plastic containers have increased permeability to environmental air as compared to metal containers. Because air can more easily permeate plastic and enter the nutritional composition matrix, an increase in oxidation can occur in the nutritional composition.

The reduction in pH of the nutritional liquid over time due to oxidation as described above can have numerous detrimental effects on the nutritional liquid inside the package including: (1) increasing the release of bound minerals, which in ionic form can compromise stability of the nutritional liquid due to precipitation; (2) increasing the amount of catalytic oxidation, particularly of iron and copper species; (3) increasing the amount of protein precipitation; and (4) increasing vitamin C destabilization. Any one of these unwanted effects can significantly reduce the commercial acceptability of the nutritional liquid.

There is therefore a need for stable nutritional liquids, such as stable protein or milk-based liquids or emulsions, that can be retort or aseptically sterilized and packaged in plastic containers or packages and that are stable and resistant to a reduction in pH over time.

## SUMMARY OF THE DISCLOSURE

The present disclosure is directed to packaged nutritional compositions comprising a plastic package and a nutritional liquid contained therein, the nutritional liquid comprising beta-hydroxy-beta-methylbutyrate and at least one of fat, and protein. The present disclosure is also directed to packaged nutritional compositions as per appended claims 2-12.

The present disclosure is further directed to a method of preparing a pH-stable nutritional liquid in a plastic package, the method comprising combining a fat, protein, carbohydrate and beta-hydroxy-beta-methylbutyrate together to form a nutritional liquid, introducing the nutritional liquid into a plastic package, and retort sterilizing the nutritional liquid in the plastic package.

The present disclosure is further directed to a method of preparing a pH-stable nutritional liquid in a plastic package, the method comprising combining a fat, protein, carbohydrate and beta-hydroxy-beta-methylbutyrate together to form a nutritional liquid, sterilizing the nutritional liquid, sterilizing a plastic package, and introducing the sterilized nutritional liquid into the sterilized plastic package.

It has been discovered that the addition of beta-hydroxy-beta-methylbutyrate (HMB) into nutritional liquids, such as nutritional emulsions, imparts an unexpected buffering effect to the nutritional liquid such that the nutritional liquid is more resistant to pH decreases upon a shift in hydrogen ion concentration over time. This unexpected effect is advantageous in that HMB is a desirable additive to nutritional compositions and, based on the discovery now made, can now be added to nutritional liquids so that the nutritional liquids can then be packaged in plastic containers where the resulting nutritional liquid is more pH-stable due to the buffering effect of the HMB present in the liquid. Because plastic packages are inherently subject to a shift in pH over time as discussed above, the unexpected discovery of the buffering effect of HMB in a nutritional liquid is particularly useful for nutritional liquids that are retort sterilized in plastic packages or aseptically sterilized and packaged in plastic packages.

## DETAILED DESCRIPTION OF THE DISCLOSURE

The packaged nutritional compositions of the present disclosure comprise a plastic container and a nutritional liquid comprising HMB contained therein, and may also include other elements, features, or ingredients. The essential elements, features or ingredients of the nutritional liquids, as well as some of the many optional variations and additions, are described in detail hereafter.

The term "HMB" as used herein, unless otherwise specified, refers to beta-hydroxy-beta-methylbutyrate (also referred to as beta-hydroxyl-3-methyl butyric acid, beta-hydroxy isovaleric acid) or a source thereof such as a calcium salt of HMB. When the source of HMB is calcium HMB, this particular source is most typically a monohydrate so that all weights, percentages, and concentrations as used herein and directed to calcium HMB are based on the weight of calcium HMB monohydrate, unless otherwise specified.

The term "nutritional liquid" as used herein, unless otherwise specified, means formulations comprising at least one of fat, and protein, and optionally carbohydrate, which are suitable for oral administration to a human and have a drinkable viscosity at the intended administration temperature, which is most typically from about 1°C to about 25°C. In this context, a drinkable viscosity at the target temperature would typically be less than about 300 cps, more typically from about 10 cps to about 160 cps, and even more typically from about 20 cps to about 70 cps. Viscosity values as used herein, unless otherwise specified, are obtained using a Brookfield Viscometer (Model DV-II+) with a 62 spindle at the target temperature. The viscosity is measured by operating the viscometer at a spindle speed that is the highest speed possible to obtain a reading that is on scale. The measured viscosity values represent the ratio of shear stress to shear rate, expressed as dynes-second/cm$^2$, or poise, or more typically as centipoise (cps) or one hundredth of a poise.

The term "shelf stable" as used herein, unless otherwise specified, refers to a nutritional liquid that can remain commercially stable after being packaged and then stored at 18-25°C for at least about 3 months, including from about 6 months to about 24 months, and also including from about 12 months to about 18 months.

The term "nutritional emulsion" as used herein, unless otherwise specified, means nutritional liquids formulated as aqueous emulsions, including water-in-oil, oil-in-water, and complex emulsions, but most typically oil-in-water emulsions.

The terms "fat" and "oil" as used herein, unless otherwise specified, are used interchangeably to refer to lipid materials derived or processed from plants or animals. These terms also include synthetic lipid materials so long as such synthetic materials are suitable for oral administration to humans.

The term "pH-stable" as used herein, unless otherwise specified, means that the pH is resistant or at least more resistant to pH reductions due to a buffering effect of beta-hydroxy-beta-methylbutyrate.

The term "plastic" as used herein, unless otherwise specified, means food grade plastics approved by the U.S. Food and Drug Administration or other suitable regulatory group, some non-limiting examples of which include polyvinyl chlorides, polyethylene terephthalate, high density polyethylene, polypropylenes, polycarbonates, and so forth.

The terms "sterile", "sterilized" or "sterilization" as used herein, unless otherwise specified, refers to the reduction in transmissible agents such as fungi, bacteria, viruses, spore forms, and so forth, in food or on food grade surfaces to the extent necessary to render such foods suitable for human consumption. Sterilization processes may include various techniques involving the application of heat, peroxide or other chemicals, irradiation, high pressure, filtration, or combinations or variations thereof.

All percentages, parts and ratios as used herein, are by weight of the total composition, unless otherwise specified. All such weights as they pertain to listed ingredients are based on the active level and, therefore, do not include solvents or byproducts that may be included in commercially available materials, unless otherwise specified.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The various embodiments of the nutritional compositions of the present disclosure may also be substantially free of any optional or selected essential ingredient or feature described herein, provided that the remaining nutritional compositions still contain all of the required ingredients or features as described herein. In this context, and unless otherwise specified, the term "substantially free" means that the selected composition contains less than a functional amount of the optional ingredient, typically less than about 0.5% by weight, including less than about 0.1%, and also including zero percent, by weight of such optional or selected essential ingredient.

The nutritional liquids and corresponding manufacturing methods of the present disclosure can comprise, consist of, or consist essentially of the essential elements and features of the disclosure as described herein, as well as any additional or optional ingredients, features, or elements described herein or otherwise useful in nutritional applications.

## Product Form

The nutritional liquids of the present disclosure comprise at least one of fat, and protein, and optionally carbohydrate, which are suitable for oral administration to a human and have a drinkable viscosity at the intended administration temperature. These compositions are most typically formulated as emulsions such as oil-in-water, water-in-oil, or complex aqueous emulsions, and even more typically as oil-in-water emulsions having a continuous aqueous phase and a discontinuous oil phase. The nutritional liquids may be shelf-stable.

The nutritional liquids may also be characterized as ready-to-feed or ready-to-drink liquids, which means that the liquids are packaged in liquid form and are suitable for consumption as such immediately upon removal from the closed plastic container holding the liquid. In other words, the present disclosure does not contemplate nutritional powders or other compositions that are formulated or otherwise reconstituted and are required to be used within 24-72 hours following formulation or reconstitution.

Although the nutritional liquids are most typically in the form of shelf stable emulsions, these liquids may also be formulated as non-emulsions such as solutions, suspensions (suspended solids), gels and so forth. These nutritional liquids may also be formulated as non-shelf stable products requiring refrigeration to maintain an extended shelf life.

The nutritional liquids typically contain up to about 95% by weight of water, including from about 50% to about 95%, also including from about 60% to about 90%, and also including from about 70% to about 85%, of water by weight of the nutritional liquids.

The nutritional liquids may be formulated with sufficient kinds and amounts of nutrients so as to provide a sole, primary, or supplemental source of nutrition, or to provide a specialized nutritional liquid for use in individuals afflicted with specific diseases or conditions. These nutritional compositions may have a variety of product densities, but most typically have a density greater than about 1.055 g/ml, including from 1.06 g/ml to 1.12 g/ml, and also including from about 1.085 g/ml to about 1.10 g/ml.

The nutritional liquids may have a caloric density tailored to the nutritional needs of the ultimate user, although in most instances the compositions comprise from about 100 to about 500 kcal/240 ml, including from about 150 to about 350 kcal/240 ml, and also including from about 200 to about 320 kcal/240 ml. These nutritional compositions also comprise HMB as described herein, the amount of which most typically ranges from about 0.5 to about 3.0 g/240 ml, including from about 0.75 to about 2.0 g/240 ml, including about 1.5 g/240 ml.

The nutritional liquids may have a pH ranging from about 3.5 to about 8, but are most advantageously in a range of from about 4.5 to about 7.5, including from about 5.5 to about 7.3, including from about 6.2 to about 7.2.

Although the serving size for the nutritional liquids can vary depending upon a number of variables, a typical serving size ranges from about 100 to about 300 ml, including from about 150 to about 250ml, including from about 190 ml to about 240 ml.

**Beta-Hydroxy-Beta-Methylbutyrate (HMB)**

The nutritional liquids comprise HMB or any source thereof that is suitable for use in an oral nutritional product and is otherwise compatible with the essential elements or features of the nutritional liquids.

The nutritional liquids most suitably comprise a calcium salt of HMB, which calcium salt is most typically in a monohydrate form. Although calcium HMB or calcium HMB monohydrate is the preferred source of HMB for use herein, other suitable sources may include HMB as the free acid, other salt forms including an anhydrous salt, an ester, a lactone, or other product forms that otherwise provide a bioavailable form of HMB from the nutritional liquid. Non-limiting examples of suitable salts of HMB for use herein include HMB salts, hydrated or anhydrous, of sodium, potassium, magnesium, chromium, calcium, or other non-toxic salt form. Calcium HMB monohydrate is preferred and is commercially available from Technical Sourcing International (TSI) of Salt Lake City, Utah.

The concentration of HMB, including the concentration of calcium HMB and or calcium HMB monohydrate when such are used as the HMB source herein, in the nutritional liquids may range up to about 10%, including from about 0.1% to about 8%, and also including from about 0.2% to about 5.0%, and also including from about 0.3% to about 3%, also including from about 0.4% to about 1.5%, and also including about 0.45% by weight of the nutritional liquid.

**Macronutrients**

The nutritional liquids comprise, in addition to HMB, at least one of fat, and protein, and optionally carbohydrate. Generally, any source of fat, protein, and carbohydrate that is known or otherwise suitable for use in nutritional products may also be suitable for use herein, provided that such macronutrients are also compatible with the essential elements of the nutritional liquids as defined herein.

Although total concentrations or amounts of the fat, protein, and carbohydrates may vary depending upon the nutritional needs of the intended user, such concentrations or amounts most typically fall within one of the following embodied ranges, inclusive of any other fat, protein, and or carbohydrate ingredients as described herein.

Carbohydrate concentrations most typically range from about 5% to about 40%, including from about 7% to about 30%, including from about 10% to about 25%, by weight of the nutritional emulsion; fat concentrations most typically range from about 1% to about 30%, including from about 2% to about 15%, and also including from about 4% to about 10%, by weight of the nutritional emulsion; and protein concentrations most typically range from about 0.5% to about 30%, including from about 1% to about 15%, and also including from about 2% to about 10%, by weight of the nutritional liquids.

The level or amount of carbohydrates, fats, and or proteins in the nutritional liquids may also be characterized in addition to or in the alternative as a percentage of total calories in the nutritional compositions as set forth in the following table.

| Nutrient (% Calories) | Embodiment A | Embodiment B | Embodiment C |
|---|---|---|---|
| Carbohydrate | 1-98 | 10-75 | 30-50 |
| **Fat** | 1-98 | 20-85 | 35-55 |
| **Protein** | 1-98 | 5-70 | 15-35 |

Non-limiting examples of suitable fats or sources thereof for use in the nutritional liquids described herein include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, and combinations thereof.

Non-limiting examples of suitable carbohydrates or sources thereof for use in the nutritional liquids described herein may include maltodextrin, hydrolyzed or modified starch or cornstarch, glucose polymers, corn syrup, corn syrup solids, rice-derived carbohydrates, glucose, fructose, lactose, high fructose corn syrup, honey, sugar alcohols (e.g., maltitol, erythritol, sorbitol), and combinations thereof.

Non-limiting examples of suitable protein or sources thereof for use in the nutritional liquids include hydrolyzed, partially hydrolyzed or non-hydrolyzed proteins or protein sources, which may be derived from any known or otherwise suitable source such as milk (e.g., casein, whey), animal (e.g., meat, fish), cereal (e.g., rice, corn), vegetable (e.g., soy) or combinations thereof. Non-limiting examples of such proteins include milk protein isolates, milk protein concentrates as described herein, casein protein isolates, whey protein, sodium and calcium caseinates, whole cow's milk, partially or completely defatted milk, soy protein isolates, soy protein concentrates, and so forth.

The nutritional liquids are particularly useful when formulated with a fat component as described herein since such ingredients may readily oxidize in an aqueous emulsion over time, thus generating increasing hydrogen ion concentrations over time that, absent the use of HMB or other buffering system herein, may result in a decrease in the composition pH and consequently a reduction in product stability.

## Soluble Protein

The nutritional liquids of the present disclosure may comprise selected amounts of soluble protein to improve product stability and minimize the development of bitter flavors and after taste over time.

The soluble protein may represent from about 35% to 100%, including from about 40% to about 85%, including from about 60% to about 80%, and also including from about 65% to about 75%, by weight of the total protein in the nutritional liquid. The concentration of soluble protein may range from at least about 0.5%, including from about 1% to about 26%, and also including from about 2% to about 15%, also including from about 3% to about 10%, and also including from about 4% to about 8%, by weight of the nutritional liquid.

The amount of soluble protein included in the nutritional liquids may also be characterized as a weight ratio of soluble protein to HMB, wherein the nutritional liquid includes a weight ratio of soluble protein to HMB, including calcium HMB and or calcium HMB monohydrate, of at least about 3.0, including from about 4.0 to about 12.0, also including from about 7.0 to about 11.0. and also including from about 8.0 to about 10.0.

The term "soluble protein" as used herein, unless otherwise specified, refers to those proteins having a solubility of at least about 90% as measured in accordance with a Protein Solubility Measurement Test that includes the following steps: (1) suspend the protein at 2.00% (w/w) in water; (2) stir vigorously for one hour at 20°C to form a suspension; (3) remove an aliquot of the suspension, and determine protein concentration as total protein; (4) centrifuge the suspension at 31,000 x g and at 20°C for one hour; (5) determine the protein concentration in the supernatant (the soluble protein); and (6) express the soluble protein as a percentage of the total protein.

Any soluble protein source is suitable for use herein provided that it meets the solubility requirement as defined herein, some non-limiting examples of which include sodium caseinate (>95% solubility as determined by the Protein Solubility Measurement Test), whey protein concentrate (>90% solubility as determined by the Protein Solubility Measurement Test), and combinations thereof. Non-soluble proteins may of course also be included in the nutritional emulsions.

Soluble protein suitable for use herein may also be characterized by the content of phosphoserine in the protein, wherein the soluble proteins in this context are defined as those proteins having at least about 100 mmoles, including from about 150 to 400 mmoles, including from about 200 to about 350 mmoles, and also including from about 250 to about 350 mmoles, of phosphoserine per kilogram of protein.

When the soluble protein is defined in terms of phosphoserine content, it has been found that the weight ratio of the soluble protein (with the defined phosphoserine content) to the calcium HMB may be at least about 3:1, including at least about 5:1, and also including at least about 7:1, and also including from about 9:1 to about 30:1. In this context, the proteins having the requisite content of phosphoserine are most typically in the form of monovalent caseinate salts such as sodium caseinate, potassium caseinate, and combinations thereof.

In one embodiment, the soluble protein may also be characterized by a mole ratio of monovalent caseinate phosphoserine to calcium HMB monohydrate of least about 0.2, including from about 0.2 to about 2.0, and also including from about 0.25 to 1.7.

It should be understood, however, that any phosphoserine-containing protein may be suitable for use herein provided that it has the requisite phosphoserine content and that the phosphoserine used in calculating the ratios are not bound, complexed, or otherwise attached to a polyvalent cation such as calcium or magnesium.

It should also be noted that alternative definitions as described herein for soluble proteins may include proteins that have little or no phosphoserine content, so that the soluble protein fraction of the compositions may include soluble protein with and/or without phosphoserine. The soluble protein for use herein may therefore be defined by any one or more of the soluble protein characterizations, separately or in combination.

The phosphoserine moieties within the protein may therefore be available for binding with the calcium released from the calcium HMB so that the above ratios of soluble protein to calcium HMB are the ratio of protein with phosphoserine moities that are unbound, unattached, or otherwise available to bind soluble calcium from the calcium HMB during formulation. It could be, for example, that a mixture of calcium caseinate and sodium caseinate are used in the composition, but the ratio of proteins defined by a phosphoserine content to calcium HMB is calculated based on the protein fraction from the sodium caseinate and additionally any protein from the calcium caseinate fraction that is not bound to calcium.

## Soluble Calcium Binding Capacity

The nutritional compositions of the present disclosure may include emulsion embodiments comprising a selected weight ratio of a soluble calcium binding capacity (SCBC) to the total soluble calcium in the emulsion to improve product stability and minimize the development over time of bitter flavors and after taste.

The ratio of the soluble calcium binding capacity (defined herein) to total soluble calcium of the emulsions embodiments is a weight ratio of at least about 2.3, including from about 2.3 to about 12.0, also including from about 3.0 to about 8.0, and also including from about 4.0 to about 6.5, wherein the ratio is determined in accordance with the following formulas:

$$\text{Ratio} = \text{SCBC} / [\text{soluble calcium}]$$

$$\text{SCBC} = (0.32 \times [\text{soluble citrate}] + 0.63 [\text{soluble phosphate}] + 0.013 \times [\text{soluble protein}])$$

The weight ratio of SCBC to the concentration of total soluble calcium can be adjusted to minimize the concentration of unbound calcium in the nutritional emulsion, or to minimize the weight ratio of such unbound calcium to HMB in the emulsions, to improve product stability and reduce the development over time of bitter flavors and after tastes.

## Calcium

The nutritional liquids of the present disclosure may further comprise calcium as desirable for use in developing or maintaining healthy muscle in targeted individuals. Some or all of the calcium may be provided when calcium HMB or calcium HMB monohydrate is used as the HMB source. Any other calcium source, however, may be used provided that such other source is compatible with the essential elements of the nutritional liquids.

The concentration of calcium in the nutritional liquids may exceed about 10 mg/L, and may also include concentrations of from about 25 mg/L to about 3000 mg/L, also including from about 50 mg/L to about 500 mg/L, and also including from about 100 mg/L to about 300 mg/L.

To minimize the taste and stability issues in the emulsion embodiments hereof, the calcium may be formulated so as to minimize the extent to which the calcium is solubilized in the emulsions. As such, solubilized calcium concentrations in the emulsion embodiments may be less than about 900 mg/L, including less than about 700 mg/L, also including from about 500 mg/L to about 700 mg/L, and also including from about 400 mg/L to about 600 mg/L. In this context, the term "solubilized calcium" refers to supernatant calcium in the nutritional liquids as measured at 20°C.

The calcium in the liquids may also be characterized by a ratio (on an equivalents basis) of solubilized citrate to solubilized calcium of not more than 5.0, including not more than 4.0, also including not more than 3.0, and also including from about 0.8 to about 3.0. In this context, the terms "solubilized citrate" and "solubilized calcium" refers to the equivalents of citrate and calcium cations, respectively, present in the supernatants of nutritional liquids as measured at 20°C.

The calcium component of the nutritional liquids may also be characterized by a solubilized calcium level that represents less than 900 mg/L, including less than 700 mg/L, and also including less than 600 mg/L, and also including from 400 mg/L to 700 mg/L of the nutritional emulsion, wherein the weight ratio of calcium HMB or its monohydrate form to the solubilized calcium ranges from about 6 to about 15, including from about 6 to about 12, also including from about 6 to about 10, and also including from about 6 to about 8.

## Vitamin D

The nutritional compositions of the present disclosure may further comprise vitamin D to help maintain healthy muscle in the targeted user. Vitamin D forms include Vitamin D2 (ergocalciferol) and Vitamin D3 (cholecalciferol) or other forms suitable for use in a nutritional product.

The amount of Vitamin D in the nutritional liquid most typically ranges up to about 1000 IU, more typically from about 10 to about 600 IU, and more typically from about 50 to 400 IU per serving.

**Optional Ingredients**

The nutritional liquids may further comprise other optional ingredients that may modify the physical, chemical, hedonic or processing characteristics of the products or serve as pharmaceutical or additional nutritional components when used in the targeted population. Many such optional ingredients are known or otherwise suitable for use in other nutritional products and may also be used in the nutritional liquids described herein, provided that such optional ingredients are safe and effective for oral administration and are compatible with the essential and other ingredients in the selected product form.

Non-limiting examples of such optional ingredients include preservatives, antioxidants, emulsifying agents, additional buffers, pharmaceutical actives, additional nutrients as described herein, colorants, flavors, thickening agents and stabilizers, and so forth.

The nutritional liquids may further comprise vitamins or related nutrients, non-limiting examples of which include vitamin A, vitamin E, vitamin K, thiamine, riboflavin, pyridoxine, vitamin B12, carotenoids, niacin, folic acid, pantothenic acid, biotin, vitamin C, choline, inositol, salts, and derivatives thereof, and combinations thereof.

The nutritional liquids may further comprise minerals, non-limiting examples of which include phosphorus, magnesium, iron, zinc, manganese, copper, sodium, potassium, molybdenum, chromium, selenium, chloride, and combinations thereof.

The nutritional liquids may also include one or more masking agents to reduce or otherwise obscure the development of residual bitter flavors and after taste in the liquids over time. Suitable masking agents include natural and artificial sweeteners, sodium sources such as sodium chloride, and hydrocolloids, such as guar gum, xanthan gum, carrageenan, gellan gum, and combinations thereof. The amount of masking agents in the nutritional liquids may vary depending upon the particular masking agent selected, other ingredients in the formulation, and other formulation or product target variables. Such amounts, however, most typically range from at least about 0.1%, including from about 0.15% to about 3.0%, and also including from about 0.18% to about 2.5%, by weight of the nutritional liquid.

**Plastic Package**

The nutritional liquids of the present disclosure are contained within a plastic package suitable for use with nutritional products or food. The plastic package should be made from food grade plastics approved by the U.S. Food and Drug Administration or other suitable regulatory group.

The packaged nutritional liquids are sterile formulations, which mean the packaged liquids have been treated or otherwise processed to reduce the amount or concentration of transmissible agents such as fungi, bacteria, viruses, spore forms, and so forth, to the extent necessary to render such packaged liquids suitable for oral consumption by humans. Sterilization processes may include various techniques involving the application of heat, chemicals, irradiation, high pressure, filtration, or combinations or variations thereof.

The plastic packaged liquids are closed systems comprising a removeable closure positioned over an opening within the package through which the liquid can be extracted prior to or during consumption during shelf-life. These plastic packages may be single or multi dose containers and may or may not have a sealing member, such as a thin foil sealing member located over the opening. The plastic container may be capable of withstanding sterilization processing, including aseptic sterilization processes, retort sterilization processes, or both processes. The plastic package may have a reclosable cap.

The plastic container, which in some embodiments may be an extruded plastic container, may comprise a single layer of plastic, or may comprise a plurality of layers of plastic that may or may not have an intermediate layer. One suitable plastic material is high-density polyethylene. A suitable intermediate layer is ethylene vinyl alcohol. In one specific embodiment, the plastic container is an eight ounce multi-layer plastic bottle with a foil seal and a recloseable cap, wherein the multilayer bottle comprises two layers of high density polyethylene with an intermediate layer of ethylene vinyl alcohol. In another embodiment, the plastic container is a 32 ounce single or multi-layer plastic bottle with a foil seal and a recloseable cap.

The plastic container or package used with the nutritional liquids described herein is generally sized and configured to minimize the headspace volume present therein. Because oxygen from air in the headspace can cause unwanted oxidation of various components of the nutritional liquid, it is generally preferred to limit the headspace volume and hence the amount of oxygen present in the plastic package. In one embodiment, the plastic package or container includes less than about 13 cubic centimeters of headspace. In another embodiment, the plastic package includes less than about 10 cubic centimeters of headspace.

**Method of Use**

The nutritional liquids described herein are useful to provide supplement, primary, or sole sources of nutrition, and/or to provide individuals one or more benefits as described herein. In accordance with such methods, the liquids may be administered orally as needed to provide the desired level of nutrition, most typically in the form of one to two servings daily, in one or two or more divided doses daily, e.g., serving sizes typically ranging from about 100 to about 300 ml, including from about 150 to about 250 ml, including from about 190 ml to about 240 ml, wherein each serving contains from about 0.4 to about 3.0 g, including from about 0.75 to about 2.0 g, including about 1.5 g, of calcium HMB per serving.

Such methods are further directed to provide the individual upon administration of such products, most typically after daily use over an extended period of time of from about 1 to about 6 months, including from about 1 to about 3 months, one or more of 1) to support maintenance of lean body mass, 2) to support of strength and/or muscle strength, 3) to decrease protein breakdown and damage of muscle cells, and 4) to help with muscle recovery following exercise or other trauma, and 5) to reduce muscle protein breakdown following exercise.

Such methods are also helpful to achieve one or more of 1) to maintain and support lean body mass in elderly with sarcopenia, 2) to provide nutrition to support an active and independent lifestyle in individuals, especially in the elderly, 3) to support recovery of muscle strength. 4) to help rebuild muscle and regain strength, and 5) to improve strength, including muscle strength, and mobility.

## Methods of Manufacture

The nutritional liquids may be manufactured by any known or otherwise suitable method for making nutritional emulsions or other nutritional liquids, most typically for making nutritional aqueous emulsions or milk based emulsions.

In one suitable manufacturing process, for example, at least three separate slurries are prepared, including a protein-in-fat (PIF) slurry, a carbohydrate-mineral (CHO-MIN) slurry, and a protein-in-water (PIW) slurry. The PIF slurry is formed by heating and mixing the selected oils (e.g., canola oil, corn oil, etc.) and then adding an emulsifier (e.g., lecithin), fat soluble vitamins, and a portion of the total protein (e.g., milk protein concentrate, etc.) with continued heat and agitation. The CHO-MIN slurry is formed by adding with heated agitation to water: minerals (e.g., potassium citrate, dipotassium phosphate, sodium citrate, etc.), trace and ultra trace minerals (TM/UTM premix), thickening or suspending agents (e.g. Avicel, gellan, carrageenan), and calcium HMB or other HMB source. The resulting CHO-MIN slurry is held for 10 minutes with continued heat and agitation before adding additional minerals (e.g., potassium chloride, magnesium carbonate, potassium iodide, etc.) and/or carbohydrates (e.g., frucotooligosaccharide, sucrose, corn syrup, etc.). The PIW slurry is then formed by mixing with heat and agitation the remaining protein (e.g., sodium caseineate, soy protein concentrate, etc.) into water.

The resulting slurries are then blended together with heated agitation and the pH adjusted to the desired range, typically 6.6-7.0, after which the composition is subjected to high-temperature short-time (HTST) processing during which the composition is heat treated, emulsified and homogenized, and then allowed to cool. Water soluble vitamins and ascorbic acid are added, the pH is adjusted to the desired range if necessary, flavors are added, and water is added to achieve the desired total solid level. The composition is then aseptically packaged to form an aseptically packaged nutritional emulsion, or the composition is added to retort stable containers and then subjected to retort sterilization to form retort sterilized nutritional emulsions or liquids.

In a retort sterilization method, for example, the nutritional liquid or emulsion may be preheated and then filled into a clean container, hermetically sealed, and placed in a steam chamber and sterilized, normally at about 121°C for about 15 to about 45 minutes. The batch is then cooled and the retort filled with a new batch. Because sterilization takes place after filling, the need for aseptic handling is eliminated, although heat resistant plastic (or another heat resistant material) must be used due to the high temperatures involved. In one specific retort sterilization embodiment, a hydrostatic tower method is utilized and includes conveying slowly the sealed containers through successive heating and cooling zones in a sterilizer. The zones are dimensioned to correspond to the required temperatures and holding times in the various treatment stages.

In an aseptic sterilization method, the nutritional liquid or emulsion may be sterilized and a container is separately sterilized. The nutritional liquid may be sterilized utilizing a heating process, for example. The container may be sterilized by spraying the interior wall of the container with hydrogen peroxide and then drying the interior wall. Once the container and the nutritional liquid have both been sterilized, the nutritional liquid is introduced into the container in a clean room environment and the container sealed.

Because aseptic sterilization generally may require the use of hydrogen peroxide as a sterilizing agent on the interior of the container, aseptically-treated nutritional liquids or emulsions packaged in aseptically sterilized containers can be subject to a change in pH over time as there is generally residual hydrogen peroxide on the interior walls of the aseptically-treated container which can enter into the liquid and emulsion and cause changes in pH. As such, it is particularly beneficial to introduce HMB into the nutritional liquids or emulsions as described herein to help buffer the liquid or emulsion and protect against unwanted shifts in pH in the product over time.

Other manufacturing processes, techniques, and variations of the described processes may be used in preparing the nutritional liquids or emulsions.

**EXAMPLES**

The following examples illustrate specific embodiments and/or features of the nutritional liquids of the present disclosure. The examples are given solely for the purpose of illustration and are not to be construed as limitations of the present disclosure, as many variations thereof are possible. All exemplified amounts are weight percentages based upon the total weight of the composition, unless otherwise specified.

**Example 1**

This illustrates the buffering effect of HMB in reconstituted PediaSure® powder (a nutritional emulsion). Known quantities of dilute hydrochloric acid are added at room temperature to a control sample of reconstituted PediaSure® powder (Abbott Laboratories, Columbus Ohio) (no HMB) and to a sample of reconstituted PediaSure® powder wherein the powder is fortified with HMB at 5.17 grams per kilogram of reconstituted powder. The HMB used to fortify the HMB-containing sample is prepared via a cation exchange removal of calcium from Calcium HMB monohydrate. Prior to the addition of the free HMB to the samples, its pH is adjusted to 6.7 with sodium hydroxide. An equimolar amount of sodium is added as sodium chloride to the control sample. With continuous stirring, the pH of each sample is measured one minute after the addition of the hydrochloric acid. From the pH reading, the hydrogen ion concentration (H+) is calculated. The results are shown in the table below:

| HCl Added (mmol/Kg) | pH PediaSure Powder w/o HMB | [H+] nmoles/kg PediaSure Powder w/o HMB | pH PediaSure Powder w/ HMB | [H+] nmoles/kg PediaSure Powder w/ HMB |
|---|---|---|---|---|
| 0 | 6.66 | 218 | 6.71 | 194 |
| 0.40 | 6.59 | 256 | 6.65 | 228 |
| 0.80 | 6.53 | 294 | 6.60 | 251 |
| 1.20 | 6.46 | 346 | 6.54 | 288 |
| 1.60 | 6.40 | 397 | 6.48 | 330 |
| 2.00 | 6.34 | 456 | 6.43 | 371 |
| 2.40 | 6.28 | 523 | 6.38 | 416 |
| 2.80 | 6.23 | 587 | 6.32 | 477 |
| 3.20 | 6.17 | 674 | 6.27 | 536 |
| 3.60 | 6.11 | 774 | 6.23 | 587 |
| 4.00 | 6.06 | 869 | 6.18 | 659 |
| Change | -0.60 | +651 | -.053 | +465 |

The data in the above table show a measurable buffering effect associated with the presence of HMB in a nutritional liquid. The overall reduction in pH for the sample including the HMB is less than the reduction in pH for the sample not including HMB. Also, the [H+] increase is less in the sample including the HMB as compared to the sample not including HMB. As such, HMB provides a buffering effect in nutritional liquids.

**Example 2**

This illustrates the buffering effect of HMB in reconstituted PediaSure® powder (a nutritional emulsion). A known quantity of hydrogen peroxide (1.32 mg/kg of reconstituted powder) is added to a control sample of reconstituted PediaSure® powder (no HMB) and to a sample of reconstituted PediaSure® powder wherein the powder is fortified with HMB at 5.17 grams per kilogram of reconstituted powder. The HMB used to fortify the sample including the HMB is prepared via a cation exchange removal of calcium from calcium HMB monohydrate. Prior to the addition of the free HMB to the samples, its pH is adjusted to 6.7 with sodium hydroxide. An equimolar amount of sodium is added as sodium chloride to the control sample. With continuous stirring, the pH of each sample is measured after one hour at room temperature and the [H+] concentrations calculated from the pH values. The results are shown in table below:

| Time After H₂O₂ Addition | pH PediaSure Powder w/o HMB | [H+], nmoles/kg, PediaSure Powder w/o HMB | pH PediaSure Powder w/ HMB | [H+], nmoles/kg, PediaSure Powder w/ HMB |
|---|---|---|---|---|
| 0-Time | 6.64 | 228 | 6.68 | 208 |
| 1 Hour | 6.55 | 281 | 6.61 | 245 |
| Change | -0.09 | +53 | -0.07 | +37 |

The data in the above table show a measurable buffering effect associated with the presence of HMB in the nutritional emulsion. The overall reduction in pH for the sample including the HMB is less than the reduction in pH for the sample not including HMB. Also, the [H+] increase is less in the sample including the HMB as compared to the sample not including HMB. As such, HMB provides a buffering effect in nutritional liquids.

### Example 3

This illustrates the buffering effect of HMB in a ready-to-drink liquid as a nutritional emulsion. The buffering capacity of commercially available Ensure® Plus (Sample #1) (Abbott Laboratories, Columbus, Ohio) and Sample #2 (liquid nutritional emulsion based on Ensure® Plus and including 6.5 grams of calcium HMB per kilogram of emulsion and 2.380 grams (g) of phosphate per kg of emulsion) are compared via hydrochloric acid titration and sodium hydroxide titration. The results are shown in table below:

| Acid or Base Added | Sample #1 (No HMB) | Sample #2 (With HMB) |
|---|---|---|
| HCl (mmoles) required to lower pH of 100 mL from 6.0 to 3.0 | 13.9 | 21.0 |
| NaOH (mmoles) required to raise pH of 100 mL from 7.0 to 11.0 | 9.62 | 9.04 |

As shown in the above table, Sample #2 including the calcium HMB is significantly more resistant to pH decrease than is Sample # 1. This data show that HMB imparts a selective buffering effect to the nutritional liquid by resisting pH decreases (via acid addition) more than pH increases (via NaOH addition). This characteristic is particularly useful in nutritional emulsions and other nutritional liquids that, over time, are more prone to pH reductions and the product instability that arises therefrom.

The pH data of Examples 1, 2, and 3 shows that when HMB is present in the nutritional liquids, it provides a buffering effect such that the nutritional liquid is more resistant to pH decreases upon addition of acids. This discovery is particularly useful when formulating nutritional liquids that are packaged in plastic containers. Because plastic containers, and especially plastic containers that are aseptically treated with a hydrogen peroxide solution, are prone to pH decreases over time, the addition of HMB into the nutritional liquid provides not only a nutritional benefit, but also a buffering effect that protects the nutritional liquid from the detrimental effects associated with a decrease in pH in the nutritional liquid.

### Examples: Liquid Nutritionals

The following examples illustrate some of the self-stable nutritional liquids of the present disclosure, which may be prepared in accordance with the manufacturing methods described herein, such that each exemplified composition, unless others specified, includes an aseptically processed embodiment and a retort packaged embodiment. The formulations are shelf stable nutritional liquids that are packaged in plastic containers and sterilized by either retort or aseptic sterilization processes. The compositions develop little or no bitter flavor or after taste over time and remain pH stable and physically stable during a shelf life of from 12-18 months at storage temperatures ranging from 1-25°C.

The exemplified compositions may be prepared by any known or otherwise suitable method for preparing nutritional liquids, including the methods described herein whereby the selected ingredients are combined into a separate carbohydrate-mineral slurry (CHO-MIN), a separate protein-in-water slurry (PIW), and a separate protein-in-fat slurry (PIF). For each individual slurry, the ingredients are mixed together under temperature and shear appropriate for the selected materials, after which the different slurries are combined in a blend tank, subjected to ultra high temperature treatment (UHT) and then homogenized at about 3000 psi. Vitamins, flavors and other heat-sensitive materials are then added to the homogenized mixture. The resulting mixture is diluted with water as needed to achieve the desired concentrations and density (generally about 1.085 to about 1.10 g/mL). The resulting nutritional liquid is then subjected to aseptic sterilization and packaging or retort sterilization and packaging using 240 ml recloseable plastic bottles. The packaged emulsions have a pH of from 3.5-7.5.

## Examples 4-7

Examples 4-7 illustrate nutritional emulsions of the present disclosure, the ingredients of which are listed in the table below. All ingredient amounts are listed as kg per 1000 kg batch of product, unless otherwise specified.

**Table 1: Nutritional Emulsions**

| Ingredient | Example 4 | Example 5 | Example 6 | Example 7 |
|---|---|---|---|---|
| Water | Q.S | Q.S. | Q.S. | Q.S. |
| Maltodextrin DE 9-12 | 120.0 | 120.0 | 120.0 | 120.0 |
| Sucrose | 71.38 | 71.38 | 71.38 | 71.38 |
| Milk Protein Concentrate | 18.65 | 18.65 | 18.65 | 18.65 |
| Canola Oil | 27.5 | 27.5 | 27.5 | 27.5 |
| Sodium Caseinate | 26.68 | 26.68 | 26.68 | 26.68 |
| Soy Protein Concentrate | 14.05 | 14.05 | 14.05 | 14.05 |
| Corn Oil | 15.70 | 15.70 | 15.70 | 15.70 |
| Calcium HMB monohydrate | 6.00 | 6.5 | 7.0 | 4 |
| Whey Protein Concentrate | 3.50 | 3.50 | 3.50 | 3.50 |
| Magnesium Phosphate | 1.92 | 1.92 | 1.92 | 1.92 |
| Potassium Citrate | 6.92 | 6.92 | 6.92 | 6.92 |
| Sodium Citrate | 0.903 | 0.903 | 0.903 | 0.903 |
| Lecithin | 1.50 | 1.50 | 1.50 | 1.50 |
| Sodium Tripolyphosphate | 1.06 | 1.06 | 1.06 | 1.06 |
| Potassium Phosphate dibasic | 0.730 | 0.730 | 0.730 | 0.730 |
| Potassium Chloride | 1.04 | 1.04 | 1.04 | 1.04 |
| Ascorbic Acid | 0.235 | 0.235 | 0.235 | 0.235 |
| Carrageenan | 0.150 | 0.150 | 0.150 | 0.150 |
| Potassium Hydroxide | 0.136 | 0.136 | 0.136 | 0.136 |
| TM/UTM Premix | 0.1684 | 0.1684 | 0.1684 | 0.1684 |
| Gellan Gum | 0.050 | 0.050 | 0.050 | 0.050 |
| Vitamin A,D, E Premix | 0.0758 | 0.0758 | 0.0758 | 0.0758 |
| Water sol. Vitamin premix | 0.0728 | 0.0728 | 0.0728 | 0.0728 |
| Potassium Iodide | 0.00022 | 0.00022 | 0.00022 | 0.00022 |
| Chromium Chloride | 0.000217 | 0.000217 | 0.000217 | 0.000217 i |
| Flavor | 3.3 | 3.3 | 3.3 | 3.3 |
| Features | | | | |
| Soluble protein/total protein (wt/wt) | 59% | 58% | 57% | 50% |
| Soluble protein/calcium HMB (wt/wt) | 6.2 | 5.6 | 5.1 | 7.5 |
| Solubilized calcium (wt%) | 0.045% | 0.049% | 0.053% | 0.070% |
| SCBC / Solubilized calcium (wt/wt) | 5.5 | 5.0 | 4.5 | 3.0 |

(continued)

| Features | | | | |
|---|---|---|---|---|
| Solubilized citrate/solubilized calcium (equiv) | 3.5 | 3.0 | 2.5 | 1.5 |

## Examples 8-11

These examples illustrate nutritional emulsions of the present disclosure, the ingredients of which are listed in the table below. All ingredient amounts are listed as kg per 1000 kg batch of product, unless otherwise specified.

**Table 2: Nutritional Emulsions**

| Ingredient | Example 8 | Example 9 | Example 10 | Example 11 |
|---|---|---|---|---|
| Water | Q.S | Q.S. | Q.S. | Q.S. |
| Maltodextrin DE 9-12 | 120.0 | 120.0 | 120.0 | 120.0 |
| Sucrose | 71.38 | 71.38 | 71.38 | 71.38 |
| Milk Protein Concentrate | 14.65 | 13.65 | 12.65 | 11.65 |
| Canola Oil | 27.5 | 27.5 | 27.5 | 27.5 |
| Sodium Caseinate | 30.68 | 31.68 | 32.68 | 33.68 |
| Soy Protein Concentrate | 14.05 | 14.05 | 14.05 | 14.05 |
| Corn Oil | 15.70 | 15.70 | 15.70 | 15.70 |
| Calcium HMB monohydrate | 6.00 | 6.5 | 7.0 | 7.5 |
| Whey Protein Concentrate | 3.50 | 3.50 | 3.50 | 3.50 |
| Magnesium Phosphate | 1.92 | 1.92 | 1.92 | 1.92 |
| Potassium Citrate | 6.92 | 6.92 | 6.92 | 6.92 |
| Sodium Citrate | 0.903 | 0.903 | 0.903 | 0.903 |
| Lecithin | 1.50 | 1.50 | 1.50 | 1.50 |
| Sodium Tripolyphosphate | 1.06 | 1.06 | 1.06 | 1.06 |
| Potassium Phosphate dibasic | 0.730 | 0.730 | 0.730 | 0.730 |
| Potassium Chloride | 1.04 | 1.04 | 1.04 | 1.04 |
| Ascorbic Acid | 0.235 | 0.235 | 0.235 | 0.235 |
| Carrageenan | 0.150 | 0.150 | 0.150 | 0.150 |
| Potassium Hydroxide | 0.136 | 0.136 | 0.136 | 0.136 |
| TM/UTM Premix | 0.1684 | 0.1684 | 0.1684 | 0.1684 |
| Gellan Gum | 0.050 | 0.050 | 0.050 | 0.050 |
| Vitamin A,D, E Premix | 0.0758 | 0.0758 | 0.0758 | 0.0758 |
| Water sol. Vitamin premix | 0.0728 | 0.0728 | 0.0728 | 0.0728 |
| Potassium Iodide | 0.00022 | 0.00022 | 0.00022 | 0.00022 |
| Chromium Chloride | 0.000217 | 0.000217 | 0.000217 | 0.000217 |
| Flavor | 3.3 | 3.3 | 3.3 | 3.3 |
| Features | | | | |
| Soluble protein/total protein (wt/wt) | 63% | 64% | 65% | 66% |
| Soluble protein/calcium HMB (wt/wt) | 6.6 | 6.2 | 5.8 | 5.0 |
| Solubilized calcium (wt%) | 0.045% | 0.049% | 0.053% | 0.070% |

(continued)

| Features | | | | |
|---|---|---|---|---|
| SCBC / Solubilized calcium (wt/wt) | 5.5 | 5.0 | 4.5 | 3.0 |
| Solubilized citrate/solubilized calcium (equiv) | 3.5 | 3.0 | 2.5 | 1.5 |

### Examples 12-15

These examples illustrate nutritional emulsions of the present disclosure, the ingredients of which are listed in the following table below. All ingredient amounts are listed as kilogram per 1000 kilogram batch of product, unless otherwise specified.

**Table 3: Nutritional Emulsions**

| Ingredient | Example 12 | Example 13 | Example 14 | Example 15 |
|---|---|---|---|---|
| Water | Q.S | Q.S. | Q.S. | Q.S. |
| Maltodextrin DE 9-12 | 120.0 | 120.0 | 120.0 | 120.0 |
| Sucrose | 71.38 | 71.38 | 71.38 | 71.38 |
| Milk Protein Concentrate | 0.00 | 0.00 | 8.65 | 10.65 |
| Canola Oil | 27.5 | 27.5 | 27.5 | 27.5 |
| Sodium Caseinate | 45.33 | 45.33 | 36.68 | 34.68 |
| Soy Protein Concentrate | 0.00 | 0.00 | 12.05 | 9.05 |
| Corn Oil | 15.70 | 15.70 | 15.70 | 15.70 |
| Calcium HMB monohydrate | 6.0 | 6.5 | 7.0 | 8.0 |
| Whey Protein Concentrate | 17.55 | 17.55 | 5.50 | 8.50 |
| Magnesium Phosphate | 1.92 | 1.92 | 1.92 | 1.92 |
| Potassium Citrate | 6.92 | 6.92 | 6.92 | 6.92 |
| Sodium Citrate | 0.903 | 0.903 | 0.903 | 0.903 |
| Lecithin | 1.50 | 1.50 | 1.50 | 1.50 |
| Sodium Tripolyphosphate | 1.06 | 1.06 | 1.06 | 1.06 |
| Potassium Phosphate dibasic | 0.730 | 0.730 | 0.730 | 0.730 |
| Potassium Chloride | 1.04 | 1.04 | 1.04 | 1.04 |
| Ascorbic Acid | 0.235 | 0.235 | 0.235 | 0.235 |
| Carrageenan | 0.150 | 0.150 | 0.150 | 0.150 |
| Potassium Hydroxide | 0.136 | 0.136 | 0.136 | 0.136 |
| TM/UTM Premix | 0.1684 | 0.1684 | 0.1684 | 0.1684 |
| Gellan Gum | 0.050 | 0.050 | 0.050 | 0.050 |
| Vitamin A,D, E Premix | 0.0758 | 0.0758 | 0.0758 | 0.0758 |
| Water sol. Vitamin premix | 0.0728 | 0.0728 | 0.0728 | 0.0728 |
| Potassium Iodide | 0.00022 | 0.00022 | 0.00022 | 0.00022 |
| Chromium Chloride | 0.000217 | 0.000217 | 0.000217 | 0.000217 |
| Flavor | 3.3 | 3.3 | 3.3 | 3.3 |
| Features | | | | |
| Soluble protein/total protein (wt/wt) | 94% | 93% | 71% | 73% |

(continued)

| Features | | | | |
|---|---|---|---|---|
| Soluble protein/calcium HMB (wt/wt) | 9.8 | 9.0 | 6.4 | 5.1 |
| Solubilized calcium (wt%) | 0.045% | 0.050% | 0.058% | 0.070% |
| SCBC / Solubilized calcium (wt/wt) | 10 | 8.8 | 5.9 | 3.8 |
| Solubilized citrate/solubilized calcium (equiv) | 3.8 | 3.4 | 2.9 | 1.5 |

## Examples 16-19

These examples illustrate nutritional emulsions of the present disclosure, the ingredients of which are listed in the following table below. All ingredient amounts are listed as kilogram per 1000 kilogram batch of product, unless otherwise specified.

**Table 4: Nutritional Emulsions**

| Ingredient | Example 16 | Example 17 | Example 18 | Example 19 |
|---|---|---|---|---|
| Water | Q.S | Q.S. | Q.S. | Q.S. |
| Sucrose | 96.05 | 96.05 | 96.05 | 96.05 |
| Maltodextrin DE 5 | 16.46 | 16.46 | 16.46 | 16.46 |
| Milk Protein Concentrate | 18.95 | 0.00 | 8.95 | 25.00 |
| Soy Oil | 13.31 | 13.31 | 13.31 | 13.31 |
| Fructooligosaccharides | 8.69 | 8.69 | 8.69 | 8.69 |
| Soy Protein Concentrate | 13.80 | 0.00 | 10.80 | 5.92 |
| Canola Oil | 5.32 | 5.32 | 5.32 | 5.32 |
| Sodium Caseinate | 25.64 | 58.39 | 61.39 | 28.00 |
| Corn Oil | 11.70 | 11.70 | 11.70 | 11.70 |
| Calcium HMB monohydrate | 6.70 | 7.00 | 2.50 | 5.00 |
| Dietary Fiber | 4.51 | 4.51 | 4.51 | 4.51 |
| Whey Protein Concentrate | 3.44 | 3.44 | 13.44 | 2.92 |
| Potassium Citrate | 4.48 | 4.48 | 4.48 | 4.48 |
| Flavor | 2.00 | 2.00 | 2.00 | 2.00 |
| Magnesium Phosphate | 2.75 | 2.75 | 2.75 | 2.75 |
| Lecithin | 1.50 | 1.50 | 1.50 | 1.50 |
| Di sodium Phosphate Dihyd | 0.436 | 0.436 | 0.436 | 0.436 |
| Potassium Phosphate Dibasic | 0.556 | 0.556 | 0.556 | 0.556 |
| Sodium Chloride | 0.498 | 0.498 | 0.498 | 0.498 |
| Choline Chloride | 0.480 | 0.480 | 0.480 | 0.480 |
| Ascorbic Acid | 0.465 | 0.465 | 0.465 | 0.465 |
| Carrageenan | 0.300 | 0.300 | 0.300 | 0.300 |
| Trace/Ultra Trace minerals | 0.420 | 0.420 | 0.420 | 0.420 |
| Potassium Chloride | 0.698 | 0.698 | 0.698 | 0.698 |
| Potassium Hydroxide | 0.321 | 0.321 | 0.321 | 0.321 |
| L-carnitine | 0.180 | 0.180 | 0.180 | 0.180 |

(continued)

| Ingredient | Example 16 | Example 17 | Example 18 | Example 19 |
|---|---|---|---|---|
| Water soluble Vitamin Premix | 0.07269 | 0.07269 | 0.07269 | 0.07269 |
| Vitamin DEK premix | 0.128 | 0.128 | 0.128 | 0.128 |
| Gellan Gum | 0.050 | 0.050 | 0.050 | 0.050 |
| Vitamin A Palmitate | 0.008245 | 0.008245 | 0.008245 | 0.008245 |
| Vitamin D3 | 0.000399 | 0.000399 | 0.000399 | 0.000399 |
| Potassium Iodide | 0.000194 | 0.000194 | 0.000194 | 0.000194 |
| Features | | | | |
| Soluble protein/total protein (wt/wt) | 58% | 95% | 80% | 61% |
| Soluble protein/calcium HMB (wt/wt) | 5.4 | 8.4 | 30 | 15 |
| Solubilized calcium (wt%) | 0.050% | 0.060% | 0.080% | 0.055% |
| SCBC / Solubilized calcium (wt/wt) | 4.4 | 9.7 | 8.8 | 4.9 |
| Solubilized citrate/solubilized calcium (equiv) | 1.3 | 3.1 | 2.7 | 2.9 |

**Examples 20-23**

These examples illustrate nutritional emulsions of the present disclosure, the ingredients of which are listed in the following table below. All ingredient amounts are listed as kilogram per 1000 kilogram batch of product, unless otherwise specified.

**Table 5: Nutritional Emulsions**

| Ingredient | Example 20 | Example 21 | Example 22 | Example 23 |
|---|---|---|---|---|
| Water | Q.S | Q.S. | Q.S. | Q.S. |
| Sucrose | 96.05 | 96.05 | 96.05 | 96.05 |
| Maltodextrin DE 5 | 16.46 | 16.46 | 16.46 | 16.46 |
| Milk Protein Concentrate | 24.98 | 0.00 | 25.00 | 10.00 |
| Soy Oil | 13.31 | 13.31 | 13.31 | 13.31 |
| Fructooligosaccharides | 8.69 | 8.69 | 8.69 | 8.69 |
| Soy Protein Concentrate | 13.64 | 0.00 | 5.87 | 10.64 |
| Canola Oil | 5.32 | 5.32 | 5.32 | 5.32 |
| Sodium Caseinate | 25.64 | 58.39 | 61.39 | 28.00 |
| Corn Oil | 11.70 | 11.70 | 11.70 | 11.70 |
| Calcium HMB monohydrate | 6.50 | 3.5 | 4.25 | 7.5 |
| Dietary Fiber | 4.51 | 4.51 | 4.51 | 4.51 |
| Whey Protein Concentrate | 3.40 | 17.04 | 6.87 | 6.40 |
| Potassium Citrate | 4.48 | 4.48 | 4.48 | 4.48 |
| Flavor | 2.00 | 2.00 | 2.00 | 2.00 |
| Magnesium Phosphate | 2.75 | 2.75 | 2.75 | 2.75 |
| Lecithin | 1.50 | 1.50 | 1.50 | 1.50 |
| Di sodium Phosphate Dihyd | 0.436 | 0.436 | 0.436 | 0.436 |
| Potassium Phosphate Dibasic | 0.556 | 0.556 | 0.556 | 0.556 |

(continued)

| Ingredient | Example 20 | Example 21 | Example 22 | Example 23 |
|---|---|---|---|---|
| Sodium Chloride | 0.498 | 0.498 | 0.498 | 0.498 |
| Choline Chloride | 0.480 | 0.480 | 0.480 | 0.480 |
| Ascorbic Acid | 0.465 | 0.465 | 0.465 | 0.465 |
| Carrageenan | 0.300 | 0.300 | 0.300 | 0.300 |
| Trace/Ultra Trace minerals | 0.420 | 0.420 | 0.420 | 0.420 |
| Potassium Chloride | 0.698 | 0.698 | 0.698 | 0.698 |
| Potassium Hydroxide | 0.321 | 0.321 | 0.321 | 0.321 |
| L-carnitine | 0.180 | 0.180 | 0.180 | 0.180 |
| Water soluble Vitamin Premix | 0.07269 | 0.07269 | 0.07269 | 0.07269 |
| Vitamin DEK premix | 0.128 | 0.128 | 0.128 | 0.128 |
| Gellan Gum | 0.050 | 0.050 | 0.050 | 0.050 |
| Vitamin A Palmitate | 0.008245 | 0.008245 | 0.008245 | 0.008245 |
| Vitamin D3 | 0.000399 | 0.000399 | 0.000399 | 0.000399 |
| Potassium Iodide | 0.000194 | 0.000194 | 0.000194 | 0.000194 |
| Features | | | | |
| Soluble protein/total protein (wt/wt) | 56% | 94% | 74% | 68% |
| Soluble protein/calcium HMB (wt/wt) | 5.8 | 20 | 17 | 5.0 |
| Solubilized calcium (wt%) | 0.057% | 0.085% | 0.079% | 0.060% |
| SCBC / Solubilized calcium (wt/wt) | 2.9 | 7.9 | 6.8 | 4.7 |
| Solubilized citrate/solubilized calcium (equiv) | 3.0 | 0.9 | 1.5 | 2.2 |

**Examples 24-27**

These examples illustrate clear, non-emulsion, liquids of the present disclosure, the ingredients of which are listed in the following table below. All ingredient amounts are listed as kilogram per 1000 kilogram batch of product, unless otherwise specified. The liquids have an adjusted pH of between 4.5 and 7.2.

**Table 6: Nutritional Emulsions**

| Ingredient | Example 24 | Example 25 | Example 26 | Example 27 |
|---|---|---|---|---|
| Ingredient Water | Q.S. | Q.S. | Q.S. | Q.S. |
| Sucrose | 27.5 | 27.5 | 27.5 | 27.5 |
| Calcium HMB monohydrate | 3.00 | 5.00 | 9.69 | 18.00 |
| L-Lysine Monohydrochloride | 2.26 | 2.26 | 2.26 | 2.26 |
| Flavor | 1.80 | 1.80 | 1.80 | 1.80 |
| Citric Acid | 1.03 | 1.03 | 1.03 | 1.03 |
| Ascorbic Acid | 0.504 | 0.504 | 0.504 | 0.504 |
| Malic Acid | 0.342 | 0.342 | 0.342 | 0.342 |
| Liquid Sucralose (25%) | 0.194 | 0.194 | 0.194 | 0.194 |
| Acesulfame Potassium | 0.113 | 0.113 | 0.113 | 0.113 |
| Vitamin D3 Water dispersible | 0.0242 | 0.0242 | 0.0242 | 0.0242 |

(continued)

| Ingredient | Example 24 | Example 25 | Example 26 | Example 27 |
|---|---|---|---|---|
| Colorant | 0.0012 | 0.0012 | 0.0012 | 0.0012 |

**Claims**

1. A packaged composition comprising a plastic package and a nutritional liquid contained therein, the nutritional liquid comprising beta-hydroxy-beta-methylbutyrate and at least one of fat and protein.

2. The packaged composition of claim 1 wherein the nutritional liquid is retort sterilized.

3. The packaged composition of claim 1 wherein the nutritional liquid is aseptically packaged.

4. The packaged composition of claim 1 wherein the nutritional liquid comprises from about 0.1% to about 8% beta-hydroxy-beta-methylbutyrate by weight of the nutritional liquid.

5. The packaged composition of claim 1 wherein the nutritional liquid comprises fat, carbohydrate, protein, and beta-hydroxy-beta-methylbutyrate, wherein the protein comprises from about 35% to 100% by weight of soluble protein and includes phosphoserine-containing protein having at least about 100 mmoles of phosphoserine per kilogram of phosphoserine-containing protein.

6. The packaged composition of claim 1 wherein the nutritional liquid has a weight ratio of a soluble calcium binding capacity to total soluble calcium of from about 2.3 to about 20.0.

7. The packaged composition of any one of claims 2 or 3, wherein the nutritional liquid comprises at least about 4.5 grams of beta-hydroxy-beta-methylbutyrate per kilogram of nutritional liquid, fat, protein, and carbohydrate, and wherein the protein is comprised of from about 35% to 100% soluble protein.

8. The packaged composition of claim 7 wherein the soluble protein includes phosphoserine-containing protein having at least about 100 mmoles of phosphoserine per kilogram of phosphoserine-containing protein.

9. The packaged composition of any one of claims 5, 7, or 8 wherein the soluble protein comprises at least one protein selected from the group consisting of sodium caseinate, whey protein concentrate, and combinations thereof.

10. The packaged composition of any one of claims 5, 7, 8, or 9 wherein the nutritional liquid comprises a weight ratio of soluble protein to beta-hydroxy-beta-methylbutyrate of from about 5:1 to about 12:1.

11. The packaged composition of any one of claims 1-10 wherein the plastic package contains less than about 13 cubic centimeters of headspace.

12. The packaged composition of any one of claims 1-11 wherein the plastic package is reclosable.

13. A method of preparing a pH-stable nutritional liquid in a plastic package, the method comprising:

    combining a fat, protein, carbohydrate and beta-hydroxy-beta-methylbutyrate together to form a nutritional liquid;
    introducing the nutritional liquid into a plastic package; and
    retort sterilizing the nutritional liquid in the plastic package.

14. A method of preparing a pH-stable nutritional liquid in a plastic package, the method comprising:

    combining a fat, protein, carbohydrate and beta-hydroxy-beta-methylbutyrate together to form a nutritional liquid;
    sterilizing the nutritional liquid;
    sterilizing a plastic package; and
    introducing the sterilized nutritional liquid into the sterilized plastic package.

**EP 2 528 457 B1**

**Patentansprüche**

1. Eine verpackte Zusammensetzung umfassend eine Plastikverpackung und eine Ernahrungsflussigkeit, die darin enthalten ist, wobei die Ernahrungsflussigkeit beta-Hydroxy-beta-methylbutyrat und mindestens eines von Fett und Protein umfasst.

2. Die verpackte Zusammensetzung gemäß Anspruch 1, worin die Ernahrungsflussigkeit Retorten-sterilisiert ist.

3. Die verpackte Zusammensetzung gemäß Anspruch 1, worin die Ernahrungsflussigkeit aseptisch verpackt ist.

4. Die verpackte Zusammensetzung gemäß Anspruch 1, worin die Ernahrungsflussigkeit von ungefähr 0,1% bis ungefähr 8% beta-Hydroxy-beta-methylbutyrat bezogen auf das Gewicht der Ernahrungsflussigkeit umfasst.

5. Die verpackte Zusammensetzung gemäß Anspruch 1, worin die Ernahrungsflussigkeit Fett, Kohlenhydrat, Protein, und beta-Hydroxy-beta-methylbutyrat umfasst, worin das Protein von ungefähr 35% bis 100% bezogen auf das Gewicht des löslichen Proteins umfasst und Phosphoserin-enthaltendes Protein mit mindestens ungefähr 100 mmol Phosphoserin pro Kilogramm Phosphoserin-enthaltendem Protein einschließt.

6. Die verpackte Zusammensetzung gemäß Anspruch 1, worin die Ernahrungsflussigkeit ein Gewichtsverhaltnis einer löslichen Kalziumbindungskapazitat zu gesamt löslichem Kalzium von ungefähr 2,3 bis ungefähr 20,0 hat.

7. Die verpackte Zusammensetzung gemäß irgendeinem der Ansprüche 2 oder 3, worin die Ernahrungsflussigkeit mindestens ungefähr 4,5 Gramm von beta-Hydroxy-beta-methylbutyrat pro Kilogramm Ernahrungsflussigkeit, Fett, Protein und Kohlenhydrat umfasst, und worin das Protein aus von ungefähr 35% bis 100% löslichem Protein besteht.

8. Die verpackte Zusammensetzung gemäß Anspruch 7, worin das lösliche Protein Phosphoserin-enthaltendes Protein mit mindestens ungefähr 100 mmol Phosphoserin pro Kilogramm von Phosphoserin-enthaltendem Protein einschließt.

9. Die verpackte Zusammensetzung gemäß irgendeinem der Ansprüche 5, 7 oder 8, worin das lösliche Protein mindestens ein Protein gewählt aus der Gruppe bestehend aus Natriumcaseinat, Molkenproteinkonzentrat und Kombinationen daraus umfasst.

10. Die verpackte Zusammensetzung gemäß irgendeinem der Ansprüche 5, 7, 8 oder 9, worin die Ernahrungsflussigkeit ein Gewichtsverhältnis von löslichem Protein zu beta-Hydroxy-betamethylbutyrat von ungefähr 5:1 bis ungefähr 12:1 umfasst.

11. Die verpackte Zusammensetzung gemäß irgendeinem der Ansprüche 1-10, worin die Plastikverpackung weniger als ungefähr 13 Kubikzentimeter Kopfraum enthält.

12. Die verpackte Zusammensetzung gemäß irgendeinem der Ansprüche 1-11, worin die Plastikverpackung wiederverschließbar ist.

13. Ein Verfahren zur Herstellung einer pH-stabilen Ernahrungsflussigkeit in einer Plastik-Verpackung, wobei das Verfahren folgendes umfasst:

    Vereinigen eines Fettes, Proteins, Kohlenhydrats und beta-Hydroxy-beta-methylbutyrat, um eine Ernahrungsflussigkeit zu bilden;
    Einfuhren der Ernahrungsflussigkeit in eine Plastikverpackung; und
    Retorten-Sterilisieren der Ernahrungsflussigkeit in der Plastikverpackung.

14. Ein Verfahren zur Herstellung einer pH-stabilen Ernahrungsflussigkeit in einer Plastik-Verpackung, wobei das Verfahren folgendes umfasst:

    Vereinigen eines Fettes, Proteins, Kohlenhydrats und beta-Hydroxy-beta-methylbutyrat, um eine Ernahrungsflussigkeit zu bilden;
    Sterilisieren der Ernahrungsflussigkeit;
    Sterilisieren einer Plastik-Verpackung; und

Einführen der sterilisierten Ernahrungsflussigkeit in die sterilisierte Plastik-Verpackung.

**Revendications**

1. Composition emballée comprenant un emballage en plastique et un liquide nutritionnel contenu à l'intérieur, le liquide nutritionnel comprenant du bêta-hydroxy-bêta-méthylbutyrate et au moins l'une d'une graisse et d'une protéine.

2. Composition emballée selon la revendication 1 où le liquide nutritionnel est stérilisé en vase clos.

3. Composition emballée selon la revendication 1 où le liquide nutritionnel est emballé de manière aseptique.

4. Composition emballée selon la revendication 1 où le liquide nutritionnel comprend d'environ 0,1 % à environ 8 % de bêta-hydroxybêta-méthylbutyrate en poids du liquide nutritionnel.

5. Composition emballée selon la revendication 1 où le liquide nutritionnel comprend une graisse, un glucide, une protéine et du bêta-hydroxy-bêta-méthylbutyrate, où la protéine comprend d'environ 35 % à 100 % en poids de protéine soluble et inclut une protéine contenant de la phosphosérine ayant au moins environ 100 mmoles de phosphosérine par kilogramme de protéine contenant de la phosphosérine.

6. Composition emballée selon la revendication 1 où le liquide nutritionnel a un rapport en poids d'une capacité de liaison du calcium soluble au calcium soluble total d'environ 2,3 à environ 20,0.

7. Composition emballée selon l'une quelconque des revendications 2 et 3 où le liquide nutritionnel comprend au moins environ 4,5 g de bêta-hydroxy-bêta-méthylbutyrate par kilogramme de liquide nutritionnel, une graisse, une protéine et un glucide, et où la protéine comprend d'environ 35 % à 100 % de protéine soluble.

8. Composition emballée selon la revendication 7 où la protéine soluble inclut une protéine contenant de la phosphosérine ayant au moins environ 100 mmoles de phosphosérine par kilogramme de protéine contenant de la phosphosérine.

9. Composition emballée selon l'une quelconque des revendications 5, 7 et 8 où la protéine soluble comprend au moins une protéine choisie dans le groupe consistant en le caséinate de sodium, un concentré de protéine du petit lait et leurs combinaisons.

10. Composition emballée selon l'une quelconque des revendications 5, 7, 8 et 9 où le liquide nutritionnel comprend un rapport en poids de protéine soluble au bêta-hydroxy-bêta-méthylbutyrate d'environ 5:1 à environ 12:1.

11. Composition emballée selon l'une quelconque des revendications 1-10 où l'emballage en plastique contient moins d'environ 13 centimètres cube d'espace libre.

12. Composition emballée selon l'une quelconque des revendications 1-11 où l'emballage en plastique peut être refermé.

13. Procédé de préparation d'un liquide nutritionnel à pH stable dans un emballage en plastique, le procédé comprenant :

   la combinaison d'une graisse, d'une protéine, d'un glucide et de bêta-hydroxy-bêta-méthylbutyrate ensemble pour former un liquide nutritionnel ;
   l'introduction du liquide nutritionnel dans un emballage en plastique ; et
   la stérilisation en vase clos du liquide nutritionnel dans l'emballage en plastique.

14. Procédé de préparation d'un liquide nutritionnel à pH stable dans un emballage en plastique, le procédé comprenant :

   la combinaison d'une graisse, d'une protéine, d'un glucide et de bêta-hydroxy-bêta-méthylbutyrate ensemble pour former un liquide nutritionnel ;
   la stérilisation du liquide nutritionnel ;
   la stérilisation d'un emballage en plastique ; et
   l'introduction du liquide nutritionnel stérilisé dans l'emballage en plastique stérilisé.

**REFERENCES CITED IN THE DESCRIPTION**

**Patent documents cited in the description**

- US 2005215640 A1